# EUROPEAN PATENT SPECIFICATION

(11) **EP 4 251 264 B1**
(45) Date of publication and mention of the grant of the patent: **05.03.2025**
(21) Application number: 21835161.7
(22) Date of filing: 24.11.2021
(51) Int. Cl.: A61N 1/375, A61N 1/372

(54) **HEADER ASSEMBLY FOR AN IMPLANTABLE INTRACARDIAC DEVICE AND RESPECTIVE INTRACARDIAC DEVICE**
KOPFANORDNUNG FÜR EINE IMPLANTIERBARE INTRAKARDIALE VORRICHTUNG UND ENTSPRECHENDE INTRAKARDIALE VORRICHTUNG
ENSEMBLE CALOTTE POUR UN DISPOSITIF INTRACARDIAQUE IMPLANTABLE ET LEDIT DISPOSITIF INTRACARDIAQUE

(30) Priority: 30.11.2020 US 202063119042 P; 26.02.2021 EP 21159588
(43) Date of publication of application: 04.10.2023
(73) Proprietor: BIOTRONIK SE & Co. KG, 12359 Berlin (DE)
(72) Inventor: AUSTIN, Eric, Portland, Oregon 97221 (US); HUGHES, Devan Hughes, Tualatin, Oregon 97062 (US); VON ARX, Jeffrey A., Lake Oswego, Oregon 97035 (US)
(74) Representative: Biotronik Corporate Services SE
(86) International application number: PCT/EP2021/082819
(87) International publication number: WO 2022/112327

(56) References cited:
- US-A1- 2016 175 583
- US-B2- 10 112 045
- US-B2- 10 874 851
- US-B2- 9 579 500

## Description

The present invention refers to an implantable intracardiac device, such as an implantable intracardiac pacemaker, and a header assembly therefor.

Active or passive intracardiac medical devices (IMD), for example implantable intracardiac pacemakers (also known as leadless pacemakers), are well known miniaturized medical devices which are entirely implanted into a heart's chamber, e.g. ventricle or atrium. Intracardiac pacemakers are used for patients who suffer from a bradycardia, that is if a heart that beats too slow to fulfil the physiological needs of the patient. Intracardiac pacemakers apply electrical stimulation in the form of pulses to the heart in order to generate a physiologically appropriate heartrate. Alternative or additional functions of intracardiac devices comprise providing other electrical or electromagnetic signals to the heart or its surrounding tissue, sensing electrical or electromagnetic signals or other physiological parameters of the heart and/or its surrounding tissue.

Document US 2012/0172690 A1 discloses a leadless pacemaker device which comprises a multi-piece tine fixation subassembly with a set of four remotely deployable active fixation tines at its distal end. The document further describes a fixation of the pacemaker to a patient tissue using the four tines positioned in a circular arrangement during minimally invasive surgery. The tines are configured to penetrate into and out of a patient's tissue of the heart when deployed and positioned adjacent to this tissue in order to securely fix the device and to provide a mechanical contact of an electrode to the patient's tissue within the center of the circular arrangement of the active fixation tines. The known active fixation tines further provide a forward pressure of electrode onto tissue to assure good electric electrode-tissue contact. However, the state-of-the-art pacemaker with known header geometry takes up a substantial portion of the pacemaker's length resulting in a longer overall device length, which could be limiting in applications of smaller patient anatomy or atrial placement.

Document US 9,579,500 discloses a tine portion for an implantable medical device including a hook segment and a distal segment terminated by a tissue-piercing tip, wherein the distal segment extends from the hook segment to the tip.

Document US 2016/0175583 discloses an electrical stimulation system employing wireless electrode assemblies to provide pacing therapy, defibrillation therapy, or other stimulation therapy.

Conversely, with known solution headers integrated into a device and maintaining constant device size, the header design takes space from other critical components, such as the battery or electronics module, affecting higher criticality device features, such as device longevity or therapeutic features that could have been incorporated into a larger electronics module.

Accordingly, there is the need for implantable intracardiac devices having smaller dimensions and at the same time providing a robust attachment mechanism and low manufacturing effort and costs.

The above problem is solved by the header assembly for an implantable intracardiac device in claim 1 and the implantable intracardiac device defined in claim 10.

In particular, the header assembly for an implantable intracardiac device according to the invention comprises an anchoring device that has at least one tine extending from a base ring, wherein the base ring is conically formed, i. e. has the shape of a truncated cone.

The header assembly is accommodated at the distal end of the intracardiac device as described in more detail below and provides a tine-based anchoring device or fixation comprising the at least one tine.

In one embodiment, the header assembly further comprises a header cap, a header spacer and optionally a collar. Particularly, the anchoring device is arranged between the header cap and the header spacer. The header spacer and/or the collar may have a washer-like shape, i.e. a flat hollow cylinder with a through opening having a smaller diameter than the cylinder wall. In one embodiment, the header cap is configured to receive and/or to retain a collar, particularly a washer-like collar, wherein particularly the collar is configured to elute an anti-inflammatory and/or immune-suppressive compound or composition, particularly towards the tissue of a patient that contacts the pacing electrode in a deployed state.

Particularly, the proximal end of the header assembly is formed by the header spacer, the base ring with the at least one tine extending therefrom, and the header cap and optionally the collar, wherein the mentioned elements may be arranged in this consecutive order from the proximal to the distal end of the header assembly. The header assembly may include a collar, particularly a washer-like collar, that is configured to elute an anti-inflammatory and/or immune-suppressive compound or composition, particularly towards the tissue that contacts the pacing electrode in a deployed state, and that is received or supported by the header cap.

In one embodiment, the header assembly comprises a plurality of tines, particularly three to six tines, e.g. four tines, extending from the base ring, wherein the tines preferably are evenly distributed along the whole circumference of the base ring of the anchoring device. The base ring is conically formed in such way that the distal end of the base ring has a greater diameter compared with its proximal end. The axis of the base ring preferably extends parallel to the axis of a housing of the intracardiac device or, in other words, from the proximal to the distal direction. The at least one tine or the plurality of tines provides or is configured for the fixation of the device within the heart's tissue after deployment.

Particularly, the implantable intracardiac device, for example a leadless pacemaker, has a cylindrical housing and the header assembly described above and in further embodiments below. Further, an electrode, particularly having preferably a rounded distal surface configured to contact the tissue of a patient, projects from the distal end of the housing, wherein the header assembly is arranged at and attached to the distal end of the housing of the intracardiac device such that the electrode projects through the header assembly, i.e. a respective through-going or complete opening of the header assembly. The electrode may act as a pacing electrode and/or sensing electrode for delivering and/or sensing therapeutic or physiological electrical pulses. The opening may be a central opening. The header spacer, the base ring of the anchoring device, the header cap, and optionally the collar comprise the opening, wherein the size of the opening of the spacer may be such that an electrode feedthrough or at least an electric conductor extending through the electrode feedthrough located at the proximal end of the electrode may be arranged within this opening. The cylindrical housing may comprise the electronics module having a processor and an energy source (e.g. a battery or coil (for wireless charging)) and, if applicable, a communication component such as an antenna. The processor may be adapted to process signals determined from the patient's body or received from the surrounding environment and/or to produce signals for treatment of the patient's heart. Such signals may comprise electrical stimulation in the form of pulses in order to generate a physiologically appropriate heartrate, and/or other electrical or electromagnetic signals to the heart or its surrounding tissue. Such signals are transformed and transmitted by the electronic module and may be applied by the electrode to the heart or its surrounding tissue. The electrode is electrically connected to the electronics module and the energy source. The hermetically sealed housing may comprise electrically conducting material, e.g. titanium, and may function as another electrode.

The above described header optimizes space due to the conical base ring and results in simpler and cheaper header components. In addition, the proposed header assembly allows an automated assembly, which results in less costs during manufacturing of the implantable intracardiac device. Accordingly, the axial length and volume of the header can be minimized with the header assembly according to the invention. Particularly, the base ring is conically shaped to allow for axial height reduction. This improvement allows more space for other more critical features of the device, such as the battery, which would increase device longevity. The saved space may be also allotted to the electronics module, to incorporate more therapeutic features. Conversely, for the same battery and electronics module size, a reduction in header length would allow for a reduction in overall device length. This may enable an application for smaller patients, or alternate placement within the heart, such as the right atrium.

In one embodiment, the base ring has a plurality of recesses (i.e. material cutouts, slots or other geometry such as a meander structure comprising a plurality of curved portions), for example at least two groups of two recesses arranged adjacent in circumferential direction within said base ring's distal end surface and extending from the distal side into the base ring's body. Alternatively, the plurality of recesses may extend from a proximal end surface of the base ring into the base ring body. As a further alternative, the plurality of recesses may extend both form the distal end surface and the proximal end surface of the base ring into the base ring body. In one embodiment, the length of one recess is at least 1/4 of the length of the base ring (i.e. its dimension into the axial direction). In one embodiment, the length of one recess is maximum 2/3 of the length of the base ring. In another embodiment the length of the recess may be at least 1/2 of the length of the base ring. The plurality of recess allows for strain reduction during the shape setting process.

In one embodiment, each of the at least one tine comprises an abutting section directly extending from the base ring of the anchoring device and forming a connection with the base ring and a middle section, wherein the abutting section of the tine continues the conical form of the base ring. Each of the plurality of tines terminates into the base ring tangent to the arc of the tines just below the surface of the header cap, and the base ring is contained between the header cap and the header spacer. The middle section of each tine of the plurality of tines has a curved form (e.g. circular curved) and the end section furthest from the base ring comprises a straight section that may be bent outwardly or may run parallel to the housing. Other forms of each extending tine are possible, as well. The base ring and/or the at least one tine may partially or fully consist of biocompatible material, e.g. a shape-memory and/or super-elastic material, for example nitinol.

Particularly, the base ring with the at least one tine is clamped and fixed between the header spacer (on its proximal side) and the header cap (on its distal side). In one embodiment, the header spacer comprises at least two projections or struts extending in distal direction, wherein an inner side face of each of the at least two projections form a positive connection with the conical base ring. Preferably, the inner side face of each of the at least two projections has the same inclination or slope as the outer lateral surface of the base ring and, if applicable, the at least two projections support the abutting section and optionally at least partly the middle section of each tine. The spacer prevents the base ring from making contact with the device housing or from damaging any Parylene or other coating material on the device housing. In one embodiment, each of the at least two projections of the spacer fits into a respective recess of the header cap.

The spacer as well as the header cap may comprise an electrically isolating material, for example PEEK (polyether ether ketone).

In one embodiment, the base ring and the at least one tine are integrally formed in one-piece, wherein particularly, the base ring and the at least one tine are made or formed from a tube. Making or forming may involve laser cutting. In one embodiment, the base ring and the at least one tine are laser cut from a tube made of a super-elastic and/or shape-memory material, particularly nitinol. Particularly, after initial cutting, the base ring and the at least one tine are preliminary iso diametric (i.e. the at least one tine is straight), and following initial cutting, the anchoring device made of the super-elastic and/or shape-memory material may be shape set by heating then cooling while held in the desired shape, wherein during shape setting the at least one tine is curved and the base ring is conically formed.

In one embodiment, the header cap comprises a recess at its distal end face, wherein the recess is adapted to accommodate the collar. The header cap extends over the base ring between each tine to securely attach the base ring and thereby the at least one tine to the device housing. The header cap may comprise at least two slotted inserts, or other suitable geometric cutout at its distal side or surface, to allow for a tool interface during assembly, or the option of automated assembly. In one embodiment, the at least two slotted inserts are located within the recess for the accommodation of the collar.

In one embodiment, the header cap comprises at least two radially outward projecting rim flanges, particularly two partially circumferential rim flanges, to allow engagement with the device housing to prevent axial dislodgement of the header assembly from the device. The at least two rim flanges may form a fraction of the outer circumference of the header cap, for example at its distal side.

In one embodiment, the distal end of the housing comprises a recess with at least two lateral projections (inwardly projecting rim flanges), wherein the header assembly is configured to be initially rotatable positioned within the recess and fixed by the lateral projections cooperating with respective lateral projections or the above described radially outward projecting flanges of the header cap. In other words, the outer diameter of the device housing extends past the electronics module portion and comprises at least two partially circumferential flanges projecting inwardly, allowing for engagement with the header cap. As indicated above, the header cap and the device housing comprise mating features that prevent rotational back twist once assembled.

In one embodiment, the collar comprises an electrically isolating material, for example silicone, particularly so that the electrode protruding though the through-going opening is electrically isolated against the device housing and/or the at least one tine. The collar may be glued in place or retained within the recess of the distal end face of the header cap in order to securely fix the collar to the header cap. Furthermore, the collar preferably comprises one or more anti-inflammatory and/or immune-suppressive drugs, such as for example, a dexamethasone compound such as dexamethasone sodium phosphate or dexamethasone acetate. In one embodiment, the collar comprises or essentially consists of a silicone matrix embedded with an anti-inflammatory and/or immune-suppressive compound, preferably a dexamethasone compound. Advantageously, the release rate of the anti-inflammatory and/or immune-suppressive may be controlled by the silicone matrix.

The present invention will now be described in further detail with reference to the accompanying schematic drawing, wherein
- Fig. 1: shows an embodiment of the inventive intracardiac device with the inventive header assembly in a perspective, exploded side view,
- Figs. 2 and 3: show the embodiment of Figure 1 in the assembled state, and
- Fig. 4: shows another embodiment of the inventive intracardiac device with the inventive header assembly in a perspective view.

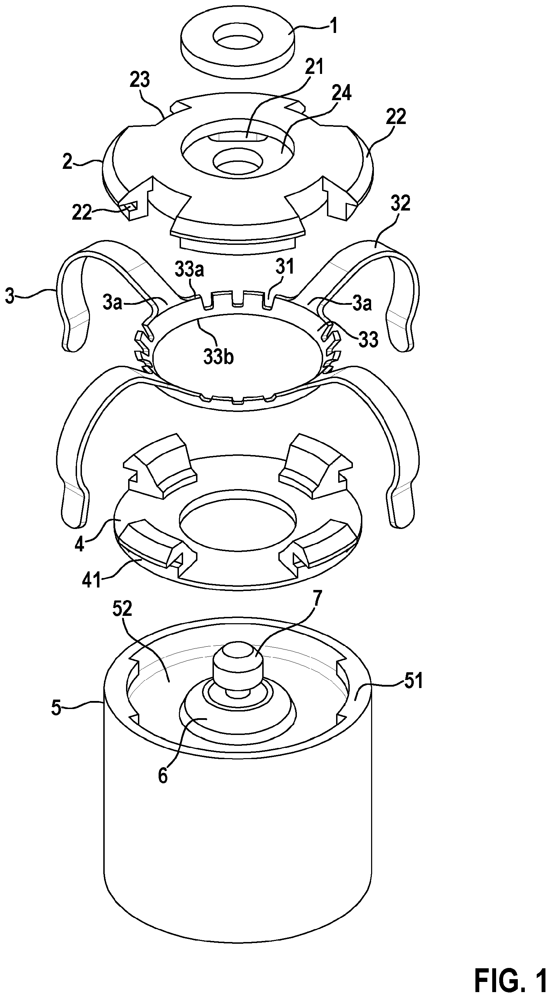

Figure 1 illustrates an exploded view of the components of the implantable intracardiac device, e.g. a leadless pacemaker, with the header assembly. The components are a circular washer-like collar 1, a substantially flatly dome-shaped header cap 2, four tines 3 formed integrally with a base ring 33, a washer-like header spacer 4, a cylindrical device housing 5, and an electrode feedthrough 6 with an electrode 7 extending therefrom in distal direction and configured to act as a pacing and/or sensing electrode. The base ring 33 is conically formed in such way that a distal end 33a of the base ring 33 has a greater diameter compared with its proximal end 33b.

The header cap 2 is designed to retain the base ring 33 with the tines 3 and the header spacer 4 by locking them into the device housing 5 with a quarter turn, engaging radially projecting rim flanges 22 of the header cap 2 with the inner lateral projections (inner rim flanges) 51 at the device housing 5, particularly at the inner surface of a distal recess 52 at the device housing 5. The projections 51 allow space for the tines 3 to engage with the header cap 2.

The header cap 2 comprises recessed slots 21 to allow for tooling interface and allow for optional automation of assembly. Further, the header cap comprises a recess 24 at its distal side into which the collar 1 is assembled. The collar 1 may be glued within this recess 24.

The tines 3 extend from the conical shaped base ring 33 which comprises four groups of three material relief recesses (slots) 31, each extending from its distal side into the ring's body to allow for shape setting of the conical ring 33. The recesses 31 are disposed over the circumference of the base ring 33 and one of the recesses 31 is arranged adjacent to another one of the recesses 31. Each tine has an abutting section (flex zone) 3a, a curved middle section and optionally a straight end section (furthest from the base ring 33) kinked outwardly. The tines 3 provide the mechanical fixation of the intracardiac device within the patient's heart after deployment and penetration of the heart's tissue such that the central electrode 7 is in mechanical and electrical contact with the inner tissue of the patient's heart within one chamber.

In this embodiment of the design of the intracardiac device, the device housing 5 is coated with a parylene provided with a deposition process to isolate the body of the housing 5 from the electrode 7.

The header spacer 4 holds the tines 3 substantially tangent to the electrode 7 and ensures electric isolation between the tines 3 and the housing 5. The header spacer 4 is designed such that four projections 41 beneath each tine 3 are shaped (e.g. rounded) to create a smooth and tangent transition from the header assembly to the device housing 5. Each projection 41 supports with its inner surface one tine 3 at its flex zone providing a positive connection and each projection 41 extends from the distal side of the spacer 4. This reduces the propensity of scar tissue formation and tissue ingrowth by minimizing geometric discontinuities. The projections 41 are accommodated within respective recesses 23 of the header cap 2, such that the base ring 33 with the tines 3 is clamped and thereby fixed between the header cap 2 and the spacer 4. The recesses 23 extend laterally from the circumference of the header cap 2 into the body of the header cap 2.

The header cap 2 may comprise notches configured to receive the tines 2 of the anchoring device as depicted in Figures 1 to 3. Alternatively, the header cap 2 and the header space 4 together form a circumferential recess in the assembled state, in which the base ring 33 of the anchoring device is arranged and retain. Preferably, the header cap 2 and the header spacer 4 are configured to allow rotatable arrangement of the anchoring device. In other words, the anchoring device may be rotated in the assembled state.

The collar 1, the header cap 2, the base ring 33 and the spacer 4 - each component comprises a central through-going opening for accommodation of the electrode 7. The diameter of the central opening of the spacer 4 is such that the electrode feedthrough 6 is located within this opening in the assembled state. The diameter of the electrode feedthrough 6 is greater than the diameter of the electrode 7.

The housing 5 of the intracardiac device contains a battery and an electronic module comprising a processor and ensures hermetically sealing of these components. These components are electrically connected to the electrode and provide the electrical stimulation of the heart or processing of electrical signals determined from the heart. Further, the housing may contain components for communication such as an antenna.

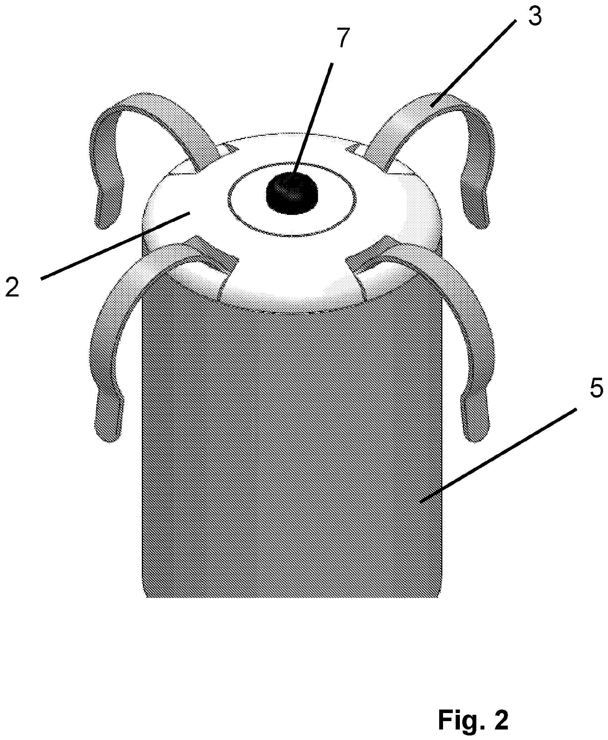

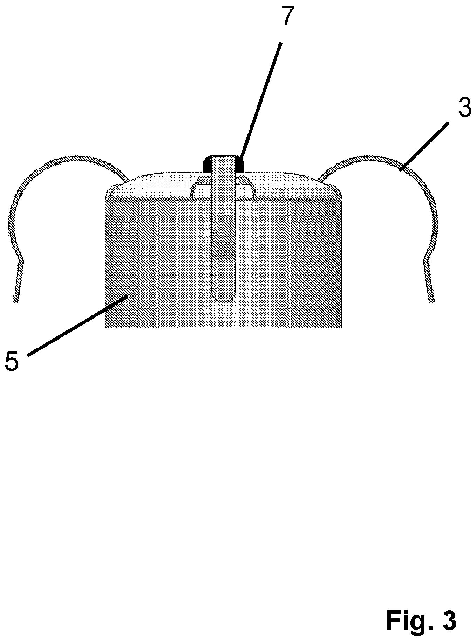

Figures 2, 3 show the intracardiac device with the header assembly according to the invention in its assembled state in a perspective view and side view, respectively.

Particularly Figure 3 illustrates the novel and inventive flat design of the header assembly according to the invention.

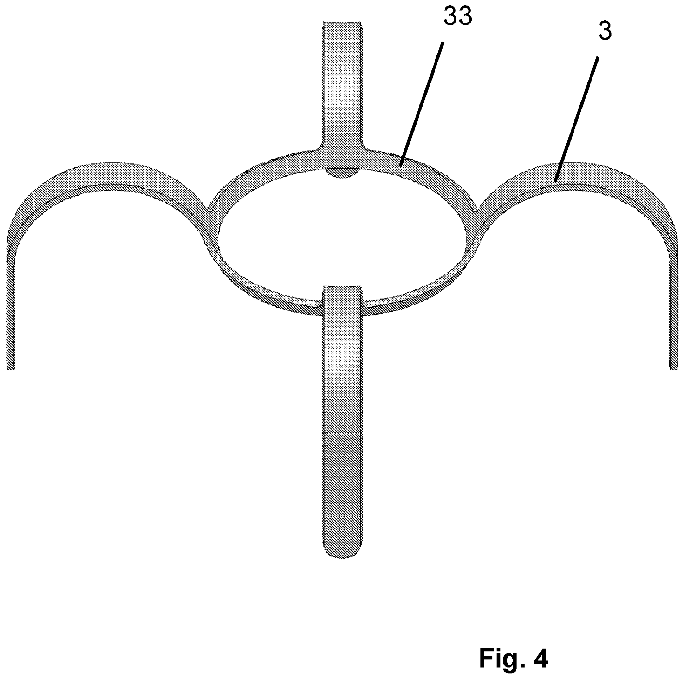

Figure 4 illustrates an alternative but also preferred embodiment of the anchoring device according to the invention. Therein, the anchoring device consists of a base ring 33 and four tines 3, particularly made of a super-elastic, shape-set material, such as nitinol, wherein preferably the base ring 33 and tines 3 are integrally formed in one-piece.

The base ring 33 is a coned-shaped, flat ribbon or sheet with a thickness being preferably equal to the thickness of the tines, from which each of the four tines 3 extends, respectively. The base ring 33 has the form of an inverted truncated, right circular, flat cone, wherein the lateral surface of the cone is inclined with respect to the rotatory axis of the cone and the anchoring device. The conical feature provides retention within the header that has been dramatically height reduced, to allow for size reduction of the overall device.

The four tines 3 particularly are evenly arranged at the base ring 33. Each of the tines 3 comprises a curved section, which extends from the base ring 33 along the inclination angle of the coned-shaped base ring 33, and a straight section extending from the curved section. The reduction in height of the conical base even further allows the shape setting to be successful without passing strain limits too high for the material.

The above intracardiac device with its inventive header assembly has reduced length in axial direction due to the conical shaped base ring 33. It is therefore less intrusive to the mechanics of the cardiac cycle and more suitable for smaller patients and challenging situations with regard to the heart (e.g. the accommodation within a heart's atrium).

## Claims

1. A header assembly for an implantable intracardiac device comprising an anchoring device having at least one tine (3) extending from a base ring (33), wherein the base ring (33) is conically formed, **characterized in that**, the base ring (33) is conically formed in such way that a distal end (33a) of the base ring (33) has a greater diameter compared with its proximal end (33b).

2. The header assembly according to claim 1, wherein the base ring (33) has a plurality of recesses (31), for example at least two groups of two recesses (31) arranged adjacent in circumferential direction within said base ring's (33) distal end surface.

3. The header assembly according to any of the previous claims, wherein each of the at least one tine (3) comprises an abutting section (3a) directly extending from the base ring (33), wherein the abutting section (3a) of the tine continues the conical form of the base ring (33).

4. The header assembly according to any of the previous claims, wherein the base ring (33) and the at least one tine (3) are formed integrally in one-piece.

5. The header assembly according to claim 4, wherein the base ring (33) and the at least one tine (3) are substantially made of a super-elastic and/or shape-memory material, particularly nitinol, wherein particularly the anchoring device is cut from a tube made of the super-elastic and/or shape-memory material, and set into the desired shape after cutting.

6. The header assembly according to any one of the previous claims further comprising a header cap (2), a header spacer (4) and optionally a collar (1), wherein particularly the anchoring device is arranged between the header cap (2) and the header spacer (4).

7. The header assembly according to claim 6, wherein the header spacer (4) comprises at least two projections (41) extending in distal direction, wherein an inner side face of each of the at least two projections (41) form a positive connection with the conical base ring (33).

8. The header assembly according to claim 7, wherein each of the at least two projections (41) of the spacer (4) fits into a respective recess (23) of the header cap (2).

9. The header assembly according to any of claims 6 to 8, wherein the header cap (2) comprises a recess (24) at its distal end face, wherein the recess (24) is configured to receive or retain the collar (1).

10. An implantable intracardiac device with a cylindrical housing (5) and the header assembly according to any of the previous claims, wherein an electrode (7) projects from the distal end of the housing (5), wherein the header assembly is arranged at the distal end of the housing (5) of the intracardiac device such that the electrode (7) projects through the header assembly.

11. The implantable intracardiac device according to claim 10, wherein the distal end of the housing (5) comprises a recess (52) with at least two lateral projections (51), wherein the header assembly is adapted to be initially rotatable positioned within the recess (52) and fixed by the lateral projections (51) cooperating with respective lateral projections (22) of the header cap (2).

## Patentansprüche

1. Kopfbaugruppe für eine implantierbare intrakardiale Vorrichtung, die eine Verankerungsvorrichtung mit mindestens einer von einem Basisring (33) vorstehenden Zinke (3) umfasst, wobei der Basisring (33) konisch ausgebildet ist, **dadurch gekennzeichnet, dass** der Basisring (33) auf solche Weise konisch ausgebildet ist, dass ein distales Ende (33a) des Basisrings (33) im Vergleich zu seinem proximalen Ende (33b) einen größeren Durchmesser aufweist.

2. Kopfbaugruppe nach Anspruch 1, wobei der Basisring (33) eine Mehrzahl von Aussparungen (31) aufweist, zum Beispiel mindestens zwei Gruppen von zwei Aussparungen (31), die innerhalb der distalen Stirnfläche des Basisrings (33) in Umfangsrichtung nebeneinander angeordnet sind.

3. Kopfbaugruppe nach einem der vorangehenden Ansprüche, wobei die mindestens eine Zinke (3) jeweils einen sich anfügenden Abschnitt (3a) umfasst, der sich direkt von dem Basisring (33) aus erstreckt, wobei der sich anfügende Abschnitt (3a) der Zinke die konische Form des Basisrings (33) fortführt.

4. Kopfbaugruppe nach einem der vorangehenden Ansprüche, wobei der Basisring (33) und die mindestens eine Zinke (3) als Einheit in einem Stück ausgebildet sind.

5. Kopfbaugruppe nach Anspruch 4, wobei der Basisring (33) und die mindestens eine Zinke (3) im Wesentlichen aus einem superelastischen und/oder Formgedächtnismaterial, insbesondere aus Nitinol, gefertigt sind, wobei die Verankerungsvorrichtung insbesondere aus einem Rohr, das aus dem superelastischen und/oder Formgedächtnismaterial gefertigt ist, geschnitten und nach dem Ausschneiden in der gewünschten Form verfestigt wird.

6. Kopfbaugruppe nach einem der vorangehenden Ansprüche, ferner eine Kopfabdeckung (2), ein Kopfdistanzstück (4) und optional einen Ring (1) umfassend, wobei die Verankerungsbaugruppe insbesondere zwischen der Kopfabdeckung (2) und dem Kopfdistanzstück (4) angeordnet ist.

7. Kopfbaugruppe nach Anspruch 6, wobei das Kopfdistanzstück (4) mindestens zwei Vorsprünge (41) umfasst, die sich in einer distalen Richtung erstrecken, wobei eine innere Seitenfläche von jedem von den mindestens zwei Vorsprüngen (41) eine formschlüssige Verbindung mit dem konischen Basisring (33) bildet.

8. Kopfbaugruppe nach Anspruch 7, wobei jeder von den mindestens zwei Vorsprüngen (41) des Distanzstücks (4) in eine jeweilige Aussparung (23) der Kopfabdeckung (2) passt.

9. Kopfbaugruppe nach einem der vorangehenden Ansprüche 6 bis 8, wobei die Kopfabdeckung (2) an ihrer distalen Stirnfläche eine Aussparung (24) umfasst, wobei die Aussparung (24) dazu eingerichtet ist, den Ring (1) aufzunehmen oder zu halten.

10. Implantierbare intrakardiale Vorrichtung mit einem zylindrischen Gehäuse (5) und der Kopfbaugruppe nach einem der vorangehenden Ansprüche, wobei eine Elektrode (7) vom distalen Ende des Gehäuses (5) vorsteht, wobei die Kopfbaugruppe am distalen Ende des Gehäuses (5) der intrakardialen Vorrichtung so angeordnet ist, dass die Elektrode (7) durch die Kopfbaugruppe vorsteht.

11. Implantierbare intrakardiale Vorrichtung nach Anspruch 10, wobei das distale Ende des Gehäuses (5) eine Aussparung (52) mit mindestens zwei lateralen Vorsprüngen (51) umfasst, wobei die Kopfbaugruppe dafür ausgelegt ist, zuerst drehbar innerhalb der Aussparung (52) positioniert und von den lateralen Vorsprüngen (51), die mit jeweiligen lateralen Vorsprüngen (22) der Kopfabdeckung (2) zusammenwirken, fixiert zu werden.

## Revendications

1. Ensemble collecteur destiné à un dispositif intracardiaque implantable comprenant un dispositif d'ancrage ayant au moins une dent (3) s'étendant depuis un anneau de base (33), dans lequel l'anneau de base (33) est façonné sous forme conique, **caractérisé en ce que** l'anneau de base (33) est façonné sous forme conique de telle sorte qu'une extrémité distale (33a) de l'anneau de base (33) a un diamètre supérieur à celui de son extrémité proximale (33b).

2. Ensemble collecteur selon la revendication 1, dans lequel l'anneau de base (33) a une pluralité d'évidements (31), par exemple au moins deux groupes de deux évidements (31) agencés de manière adjacente dans une direction circonférentielle à l'intérieur de la surface d'extrémité distale dudit anneau de base (33).

3. Ensemble collecteur selon l'une quelconque des revendications précédentes, dans lequel chacune des au moins une dent (3) comprend une section en butée (3a) s'étendant directement depuis l'anneau de base (33), dans lequel la section en butée (3a) de la dent prolonge la forme conique de l'anneau de base (33).

4. Ensemble collecteur selon l'une quelconque des revendications précédentes, dans lequel l'anneau de base (33) et l'au moins une dent (3) sont formés de manière intégrale d'une seule pièce.

5. Ensemble collecteur selon la revendication 4, dans lequel l'anneau de base (33) et l'au moins une dent (3) sont essentiellement fabriqués en un matériau super-élastique et/ou à mémoire de forme, en particulier le nitinol, dans lequel en particulier le dispositif d'ancrage est découpé dans un tube fabriqué en le matériau super-élastique et/ou à mémoire de forme, et façonné en la forme souhaitée après découpe.

6. Ensemble collecteur selon l'une quelconque des revendications précédentes comprenant en outre un capuchon de collecteur (2), un espaceur de collecteur (4) et éventuellement un collier (1), dans lequel en particulier le dispositif d'ancrage est agencé entre le capuchon de collecteur (2) et l'espaceur de collecteur (4).

7. Ensemble collecteur selon la revendication 6, dans lequel l'espaceur de collecteur (4) comprend au moins deux saillies (41) s'étendant dans la direction distale, dans lequel une face de côté intérieur de chacune des au moins deux saillies (41) forme un raccordement positif avec l'anneau de base (33) conique.

8. Ensemble collecteur selon la revendication 7, dans lequel chacune des au moins deux saillies (41) de l'espaceur (4) s'ajuste dans un évidement (23) respectif du capuchon de collecteur (2).

9. Ensemble collecteur selon l'une quelconque des revendications 6 à 8, dans lequel le capuchon de collecteur (2) comprend un évidement (24) au niveau de sa face d'extrémité distale, dans lequel l'évidement (24) est conçu pour recevoir ou retenir le collier (1).

10. Dispositif intracardiaque implantable avec un boîtier cylindrique (5) et l'ensemble collecteur selon l'une quelconque des revendications précédentes, dans lequel une électrode (7) fait saillie depuis l'extrémité distale du boîtier (5), dans lequel l'ensemble collecteur est agencé au niveau de l'extrémité distale du boîtier (5) du dispositif intracardiaque de telle sorte que l'électrode (7) fait saillie à travers l'ensemble collecteur.

11. Dispositif intracardiaque implantable selon la revendication 10, dans lequel l'extrémité distale du boîtier (5) comprend un évidement (52) avec au moins deux saillies latérales (51), dans lequel l'ensemble collecteur est adapté pour pouvoir être initialement positionné de manière rotative à l'intérieur de l'évidement (52) et fixé par les saillies latérales (51) en collaboration avec les saillies latérales (22) respectives du capuchon de collecteur (2).
